# EUROPEAN PATENT APPLICATION

(11) **EP 1 905 450 A1**
(43) Date of publication of application: **02.04.2008**
(21) Application number: 06768088.4
(22) Date of filing: 11.07.2006
(51) Int. Cl.: A61K 45/00, A61K 31/155, A61K 31/192, A61K 31/4184, A61K 31/421, A61K 31/425, A61K 31/4439, A61K 31/47, A61K 45/06, A61P 3/00, A61P 3/10, A61P 43/00

(54) **PHARMACEUTICAL COMPOSITION CONTAINING PPAR GAMMA AGONIST**

(30) Priority: 12.07.2005 JP 2005202601
(71) Applicant: Daiichi Sankyo Company, Limited, Chuo-ku Tokyo 103-8426 (JP)
(72) Inventor: OKUNO, Akira c/0 Daiichi Sankyo Company Limited, Shinagawa-ku, Tokyo 140-8710 (JP); YOSHIDA, Taishi c/0 Daiichi Sankyo Company Limited, Shinagawa-ku, Tokyo 140-8710 (JP); OGAWA, Junko c/0 Daiichi Sankyo Company Limited, Shinagawa-ku, Tokyo 140-8710 (JP)
(74) Representative: Gibson, Christian John Robert
(86) International application number: PCT/JP2006/313775
(87) International publication number: WO 2007/007757

(57) **Abstract**

[OBJECT]

To provide a highly safe method for treating diabetes that demonstrates superior effects and is capable of inhibiting adverse side effects.

[MEANS FOR SOLUTION]

A pharmaceutical containing a PPARγ agonist, and a pharmaceutical composition in combination with a biguanide agent and a PPARγ agonist.

## Description

### [TECHNICAL FIELD]

The present invention relates to a pharmaceutical composition comprising a PPARγ agonist, and to a pharmaceutical composition comprising a PPARγ agonist in combination with a biguanide agent (preferably a pharmaceutical composition for treating and/or preventing diabetes).

Moreover, the present invention relates to use of the aforementioned compound for manufacturing the aforementioned pharmaceutical composition, and to a method for preventing or treating the aforementioned disease by administering the aforementioned pharmaceutical composition to a warm-blooded animal (preferably a human).

### [BACKGROUND ART]

PPARγ agonists are known to lower blood glucose levels by improving insulin disfunction, and are administered to diabetic patients as insulin sensitizers. Examples of current commercially available PPARγ agonists include pioglitazone and rosiglitazone.

On the other hand, since biguanide agents have an action that lowers blood glucose without increasing body weight, they are commonly used as anti-diabetic agents in the US and Europe. Examples of current commercially available biguanide agents include metformin, and these are administered to diabetic patients one to three times per day. However, metformin and other biguanide agents are known to be associated with the occurrence of adverse events, such as gastrointestinal disorders (for example, malaise and indigestion) or lactic acidosis with accompanying elevated lactate levels, during clinical use, thus requiring adequate caution to be taken at the time of use thereof.

In addition, the combined use of insulin sensitizers and biguanide agents for the treatment of diabetes is already known (for example, rosiglitazone and metformin fixed dose preparation described in Patent Document 1 or 2). However, the specific effects of PPARγ agonists on blood lactate are completely unknown.
[Patent Document 1] International Publication No. WO 99/3477 pamphlet
[Patent Document 2] International Publication No. WO 2001/35941 pamphlet

### [DISCLOSURE OF THE INVENTION]

### [Problems to be Solved by the Invention]

As a result of conducting extensive studies for the purpose of developing a highly safe method for treating diabetes that demonstrates superior effects and is capable of inhibiting adverse side effects (for example, elevated lactate levels), the inventors of the present invention found that a PPARγ agonist lowers lactate levels, and that high diabetes therapeutic effects are obtained and that adverse side effects can be significantly inhibited by combining a biguanide agent and a PPARγ agonist, thereby leading to completion of the present invention.

### [Means for Solving the Problems]

The present invention is:
(1) a pharmaceutical composition for lowering blood lactate levels, comprising a PPARγ agonist as an active ingredient thereof;
(2) a pharmaceutical composition for inhibiting increases in blood lactate levels by promoting elimination of blood lactate, comprising a PPARγ agonist as an active ingredient thereof;
(3) a pharmaceutical composition for preventing accumulation of lactate in the blood by promoting elimination of blood lactate, comprising a PPARγ agonist as an active ingredient thereof;
(4) a pharmaceutical composition for inhibiting increases in blood lactate levels comprising a PPARγ agonist in combination with a biguanide agent;
(5) the pharmaceutical composition according to any one of (1) to (4) above, wherein the blood lactate is generated from the action of a biguanide agent;
(6) the pharmaceutical composition according to any one of (1) to (5) above, wherein the PPARγ agonist is pioglitazone, rosiglitazone, MCC-555, BMS-298585, AZ-242, LY-519818, R-483, 3-(2,4-dichlorobenzyl)-2-methyl-N-(pentylsulfonyl)-3H-benzimidazole-5-carboxamide, MBX-102, AMG-131, 5-[4-(6-methoxy-1-methyl-1H-benzimidazol-2-ylmethoxy)benzyl]thiazolidine-2,4-dione or pharmaceutically acceptable salt thereof;
(7) the pharmaceutical composition according to any one of (1) to (5) above, wherein the PPARγ agonist is a thiazolidinedione-based PPARγ agonist;
(8) the pharmaceutical composition according to any one of (1) to (5) above, wherein the PPARγ agonist is pioglitazone, rosiglitazone or a pharmaceutically acceptable salt thereof;
(9) the pharmaceutical composition according to any one of (1) to (5) above, wherein the PPARγ agonist is 5-[4-(6-methoxy-1-methyl-1H-benzimidazol-2-ylmethoxy)benzyl]thiazolidine-2,4-dione or a pharmaceutically acceptable salt thereof;
(10) the pharmaceutical composition according to any one of (1) to (9) above, wherein the biguanide agent is metformin, fenformin or buformin;
(11) the pharmaceutical composition according to any one of (1) to (9) above, wherein the biguanide agent is metformin;
(12) the pharmaceutical composition according to any one of (1) to (11) above for preventing or treating diabetes;
(13) use of a PPARγ agonist for manufacturing a blood lactate lowering agent;
(14) a kit for preventing adverse side effects occurring due to the action of a biguanide agent in the treatment of diabetes, wherein (a) a first component is a pharmaceutical composition comprising a PPARγ agonist as an active ingredient thereof, and (b) a second component is a pharmaceutical composition comprising a biguanide agent as an active ingredient thereof;
(15) the kit according to (14) above, wherein the adverse side effect occurring due to the action of the biguanide agent is an increase in blood lactate;
(16) the kit according to (14) or (15) above that is packaged for oral administration;
(17) use of a biguanide agent and a PPARγ agonist for manufacturing a kit for preventing adverse side effects occurring due to the action of a biguanide agent in the treatment of diabetes, wherein (a) a first component is a pharmaceutical composition comprising a PPARγ agonist as an active ingredient thereof, and (b) a second component is a pharmaceutical composition comprising a biguanide agent as an active ingredient thereof;
(18) a method for treating diabetes that alleviates adverse side effects caused by a biguanide agent, comprising: concomitant administration to a patient of (a) a pharmaceutical composition comprising a PPARγ agonist as an active ingredient thereof, and (b) a pharmaceutical composition comprising a biguanide agent as an active ingredient thereof;
(19) a method for treating diabetes that alleviates adverse side effects caused by a biguanide agent, comprising: concomitant administration to a patient of (a) a pharmaceutical composition comprising a PPARγ agonist as an active ingredient thereof, followed by (b) a pharmaceutical composition comprising a biguanide agent as an active ingredient thereof; and,
(20) the treatment method according to (18) or (19) above, wherein the adverse side effect caused by the biguanide agent is accumulation of blood lactate.

In the present invention, a "PPARγ agonist" is the generic term for a drug that improves insulin resistance and enhances insulin sensitivity by activating peroxisome proliferator activated receptors (PPAR), and is also referred to as a PPARγ agonist. Examples of PPARγ agonists include pioglitazone (preferably pioglitazone hydrochloride), rosiglitazone (preferably rosiglitazone maleate), MCC-555, BMS-298585, AZ-242, LY-519818, R-483, MBX-102 and AMG-131 (preferably para-toluenesulfonate) represented by the structural formulas indicated below: 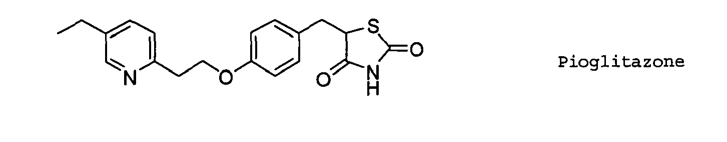 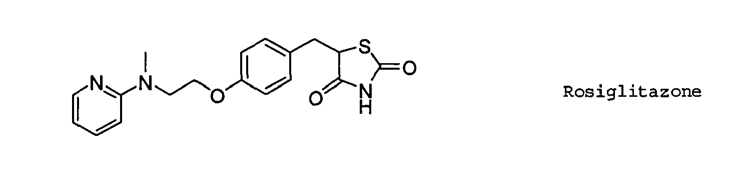 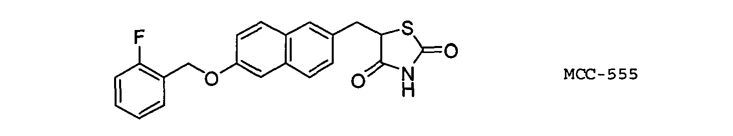 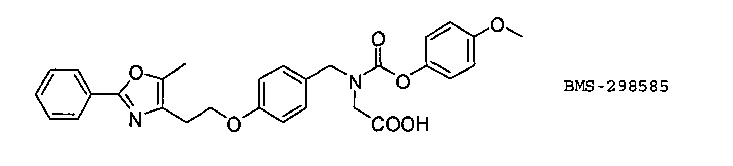 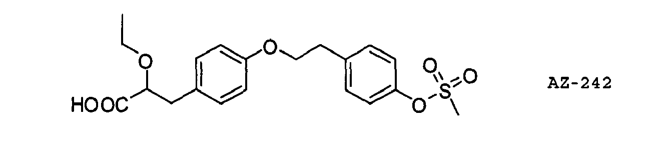 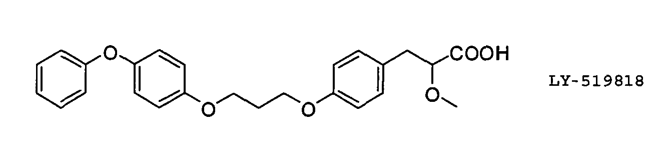 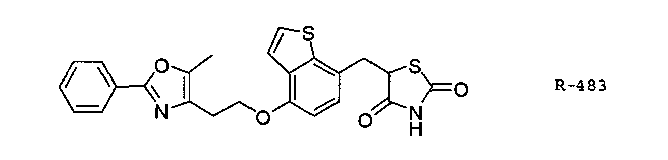 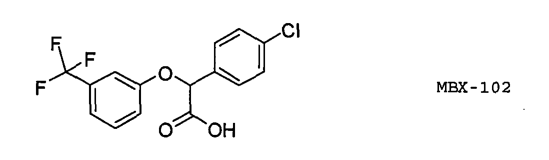 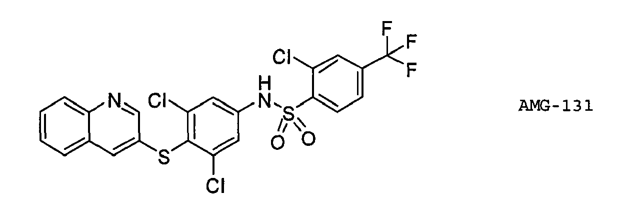 3-(2,4-dichlorobenzyl)-2-methyl-N-(pentylsulfonyl)-3H-benzimidazole-5-carboxamide (FK-614), 5-[4-(6-methoxy-1-methyl-1H-benzimidazol-2-ylmethoxy)benzyl]thiazolidine-2,4-dione and pharmaceutically acceptable salts thereof (preferably 5-[4-(6-methoxy-1-methyl-1H-benzimidazol-2-ylmethoxy)benzyl]thiazolidine-2,4-dione hydrochloride). Thiazolidinedione PPARγ agonists such as pioglitazone, rosiglitazone, 5-[4-(6-methoxy-1-methyl-1H-benzimidazol-2-ylmethoxy)benzyl]thiazolidine-2,4-dione and pharmaceutical acceptable salts thereof are preferable.

Pioglitazone is a compound described in US Patent No. 4,687,777. Rosiglitazone is a compound described in US Patent No. 5,002,953. MCC-555 is a compound described in US Patent No. 5,594,016. BMS-298585 is a compound described in International Publication No. WO 01/21602 pamphlet. AZ-242 is a compound described in International Publication No. WO 99/62872 pamphlet. LY-519818 is a compound described in International Publication No. WO 02/100813 pamphlet. 3-(2,4-dichlorobenzyl)-2-methyl-N-(pentylsulfonyl)-3H-benzimidazole-5-carboxamide (FK-614) is a compound described in US Patent No. 6,166,219. MBX-102 is a compound described in US Patent No. 6,262,118. AMG-131 is a compound described in International Publication No. WO 2001/579 pamphlet, International Publication No. WO 2001/579 pamphlet and International Publication No. WO 2005/33074 pamphlet. 5-[4-(6-methoxy-1-methyl-1H-benzimidazol-2-ylmethoxy)benzyl]thiazolidine-2,4-dione is a compound or pharmaceutically acceptable salt thereof represented by the following structure: 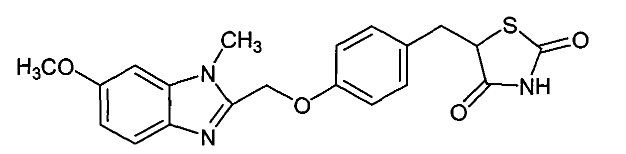 described in Japanese Patent Application (Kokai) No. Hei 9-295970, European Patent No. EP-0745600, US Patent No. 5,886,014 and International Publication No. WO 00/71540 pamphlet.

In the present invention, there are no particular limitations on the "biguanide agent" provided it is a drug having an action such as promotion of anaerobic glycolyis, enhancement of the peripheral action of insulin, inhibition of absorption of glucose from the intestinal tract or inhibition of hepatic gluconeogenesis, examples of which include 1,1-dimethylbiguanide hydrochloride (generic name: metformin), fenformin and buformin, with metformin being particularly preferable.

Biguanide agents and PPARγ agonists can be administered in the form of a fixed dose preparation. In addition, they can also be separately administered simultaneously. In addition, each preparation can be administered in sequence at a suitable interval. The allowed administration interval for achieving the effects brought about by administration of these preparations can be confirmed clinically or from animal experiments.

The pharmaceutical composition of the present invention is administered in various forms. There are no particular limitations on the administration form thereof, and the administration form is determined according to each preparation form, patient age, gender and other conditions, disease severity and the like. For example, in the case of tablets, pills, powders, granules, syrups, liquids, suspensions, emulsions, granules and capsules, they are orally administered.

Each of these types of preparations can be prepared by formulating known auxiliaries ordinarily used in known pharmaceutical fields, such as excipients, binders, disintegrants, lubricants, dissolving agents, corrigents or coating agents, with the active ingredient in accordance with ordinary methods.

For example, granules can be obtained by adding an active ingredient, stabilizer, excipient, binder, disintegrant and, as necessary, other assistants and the like, mixing with a highspeed mixer granulator and adding an aqueous solution of a binder to the resulting mixture followed by kneading. The resulting granules are then dried using a fluid bed dryer, the dried granules are forcibly passed through a screen using a crushing-granulating particle sizer, and a lubricant, disintegrant and, as necessary, other auxiliaries and the like are added and mixed with a V-type mixer followed by forming the resulting mixture into tablets or filling into capsules to produce tablet or capsule preparations, respectively.

When the pharmaceutical composition is molded into the form of tablets, a conventionally known carrier widely used in this field can be used for the carrier, examples of which include excipients such as lactose, saccharose, sodium chloride, glucose, urea, starch, calcium carbonate, kaolin, crystalline cellulose or silicic acid, binders such as water, ethanol, propanol, simple syrup, glucose solution, starch solution, gelatin solution, carboxymethyl cellulose, shellac, methyl cellulose, potassium phosphate or polyvinylpyrrolidone, disintegrants such as dried starch, sodium alginate, powdered agar, powdered laminaran, sodium bicarbonate, calcium carbonate, polyoxyethylene sorbitan fatty acid esters, sodium lauryl sulfate, monoglyceride stearate, starch or lactose, disintegration inhibitors such as saccharose, stearin, cocoa butter or hydrogenated oils, absorption promoters such as quaternary ammonium base or sodium lauryl sulfate, moisturizers such as glycerin or starch, adsorbents such as starch, lactose, kaolin, bentonite or colloidal silicic acid, and lubricants such as refined talc, stearates, powdered boric acid or polyethylene glycol.

Moreover, tablets can be in the form of ordinary coated tablets as necessary, examples of which include sugar-coated tablets, gelatin-coated tablets, enteric-coated tablets, film-coated tablets or double-layered or multilayered tablets.

When the pharmaceutical composition is molded into the form of pills, a conventionally known carrier widely used in this field can be used for the carrier, examples of which include excipients such as glucose, lactose, starch, cocoa butter, hardened vegetable oil, kaolin or talc, binders such as powdered gum Arabic, powdered tragacanth, gelatin or ethanol, and disintegrants such as laminaran or agar.

Moreover, a colorant, preservative, fragrance, flavouring, sweetener or other pharmaceutical may also be contained as necessary.

The dosage and administration ratio of each of the diabetes therapeutic agents used in the present invention can be varied over a wide range according to various conditions such as the activity of individual substances and symptoms, age or body weight of the patient.

Although there are no particular limitations on the PPARγ agonist contained in the aforementioned pharmaceutical preparations and it may be suitably selected over a wide range, the amount thereof contained is suitably 0.01 to 50% by weight and preferably 0.1 to 10% by weight of the total composition.

Although varying according to symptoms, age, body weight, drug form and the like, the adult dosage normally can have a lower limit of 0.0001 mg/kg (preferably 0.001 mg/kg and more preferably 0.01 mg/kg) per day and an upper limit of 30 mg/kg (preferably 3 mg/kg and more preferably 0.3 mg/kg) per day administered in 1 or 2 administrations.

Although there are no particular limitations on the amount of biguanide agent contained in the aforementioned preparations and the amount is suitably selected over a wide range, the amount thereof contained is suitably 1 to 70% by weight and preferably 1 to 50% by weight of the total composition.

Although varying according to symptoms, age, body weight, drug form and the like, the adult dosage normally can have a lower limit of 0.0001 mg/kg (preferably 0.001 mg/kg and more preferably 0.01 mg/kg) per day and an upper limit of 40 mg/kg (preferably 15 mg/kg and more preferably 10 mg/kg) per day administered in 1 to 3 administrations.

In the present invention, the biguanide agent and PPARγ agonist are administered at the respective dosages described above once a day or divided into multiple administrations either simultaneously or separately at different times.

### [Effects of the Invention]

According to the present invention, since high therapeutic effects can be obtained and adverse side effects can be significantly inhibited by administrating a combination of a PPARγ agonist and a biguanide agent, the pharmaceutical composition of the present invention is extremely useful in the treatment of diabetes.

### [BEST MODE FOR CARRYING OUT THE INVENTION]

The present invention is described in more detail by way of examples and the like, but the present invention is not limited thereto.

### [Examples]

The PPARγ agonist in the form of 5-{4-[(6-methoxy-1-methyl-1H-benzimidazol-2-yl)methoxy]benzyl}thiazolidine-2,4-dione hydrochloride (hereinafter referred to as Compound B) can be produced according to the method described in Japanese Patent Application (Kokai) No. Hei 9-295970, European Patent No. 0745600, US Patent No. 5,886,014 and International Publication No. WO 00/71540 pamphlet.

### <Test Example 1>

Improvement Effects by Concomitant Use of 5-{4-[(6-methoxy-1-methyl-1H-benzimidazol-2-yl)methoxy]benzyl}thiazolidine-2,4-dione hydrochloride (Compound B) for Delayed Elimination of Lactate caused by Administration of Metformin (hereinafter referred to as Compound A)

Male Zucker diabetic fatty (ZDF) rats (sold by Japan Charles River, Inc.) age 10 weeks presenting diabetes were divided into four groups consisting of a control group, a Compound A dose group (Group A), a Compound B treatment control group (Group B) and a Compound A-Compound B dose group (Group B+A). The animals of Group B and Group B+A were given free access for 1 week to Compound B mixed in powdered feed (FR-2 Powdered Laboratory Rodent Diet, Funabashi Farm Co., Ltd.) to a concentration of 0.001%. Animals of the control group and Group A were given free access to powdered feed for the same time period.

Compound A was orally administered at 300 mg/kg to the animals of Group A and Group B+A one week after the start of treatment with Compound B, while the animals of the control group and Group B were orally administered dosing solvent only. All animals were administered sodium lactate at 0.1 g/kg into the caudal vein one hour after oral administration. Blood samples were collected from the caudal vein immediately before and 5, 15, 30, 45 and 60 minutes after administration of sodium lactate, and blood lactate levels were measured using the Lactate Pro LT-1710 (Arkray, Inc.). The areas under the curve for blood lactate concentration (blood lactate AUC) were calculated from blood lactate levels from 0 to 60 minutes measured for each animal. A lower blood lactate AUC value indicates a more rapid elimination of lactate from the blood.

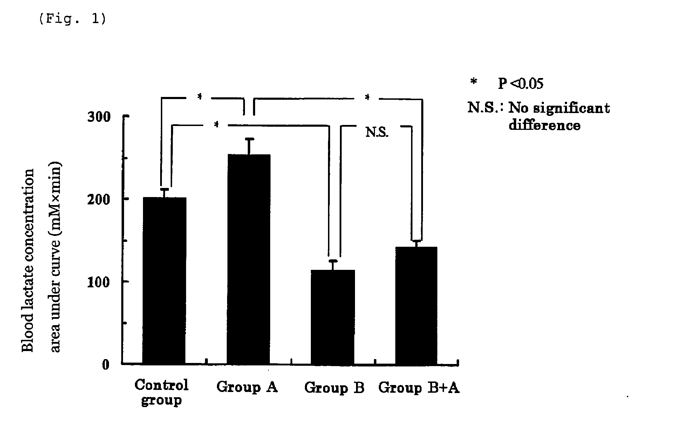

The mean values and standard errors of the blood lactate AUC values for each group were calculated and shown in Fig. 1.

According to Fig. 1, the blood lactate AUC values in Group B decreased significantly as compared with the control group (P<0.05, t-test). On the basis of this finding, treatment with Compound B alone was indicated to be effective in promoting elimination of lactate.

In addition, the blood lactate AUC values in Group A increased significantly as compared with the control group (P<0.05, t-test). In contrast, the blood lactate AUC values in Group B+A decreased significantly relative to Group A (P<0.05, t-test). On the basis of these findings, delays in elimination of lactate occurring due to administration of metformin (Compound A) were clearly demonstrated to be improved by treatment with Compound B.

Cases are known in which prominent increases in blood lactate levels and accompanying acidosis occur as a result of administration of biguanide agents, and blood lactate levels are one of the important parameters monitored during the use of biguanide agents for treating diabetes. For example, one of the modes of the mechanism of the blood glucose lowering action of metformin, a type of biguanide agent, has been reported to involve inhibition of gluconeogenesis in the liver (Diabetes Research and Clinical Practice, Vol. 19, No. 11-17, pp. 49-58, 1993), and the mechanism behind this inhibition of hepatic gluconeogenesis is believed to involve an action that inhibits uptake of alanine, a gluconeogenesis substrate, into the liver, and an action that reduces pyruvic acid, also a gluconeogenesis substrate, to lactate, thus leading to the occurrence of adverse side effects in the manner of lactic acidosis. Although the mechanism behind the increase in lactate levels during administration of metformin has yet to be made clear, an action that promotes anaerobic glycolysis with accompanying respiratory chain inhibition in mitochondria has been suggested to be a possible cause (Biochemical Journal, Vol. 348, pp. 607-614, 2000).

On the other hand, PPARγ agonists have been reported to decrease the expression of pyruvate dehydrogenase kinase that negatively regulates the activity of pyruvate dehydrogenase, which is an enzyme responsible for converting pyruvic acid to acetyl CoA during the aerobic glycolysis process (Journal of Lipid Research, Vol. 45, pp. 113-123, 2004).

Based on the above, improvement of conditions facilitating the consumption of lactate attributable to activation of pyruvate dehydrogenase as a result of treatment with PPARγ agonist is thought to be one of the reasons for a delay in lactate elimination caused by administration of biguanide agent not occurring.

Thus, since the combined use of a PPARγ agonist and a biguanide agent makes it possible to improve blood glucose control while diminishing adverse side effects associated with the use of biguanide agents (particularly elevated blood lactate levels), it is thought to be an extremely superior treatment method for diabetes patients.

### <Preparation Example>

### (1) Capsule Preparation

| | |
|---|---|
| Compound A | 250 mg |
| Compound B | 1 mg |
| Lactose | 80.2 mg |
| Carmellose sodium | 18 mg |
| Hydroxypropyl cellulose | 7.2 mg |
| Magnesium stearate | 3.6 mg |
| Total | 360 mg |

Powders of each of the components listed above can be mixed well followed by filling into capsules to produce a capsule preparation.

### (2) Tablet Preparation

| | |
|---|---|
| Compound A | 500 mg |
| Compound B | 2 mg |
| Lactose | 45.2 mg |
| Carmellose sodium | 72 mg |
| Crystalline cellulose | 72 mg |
| Hydroxypropyl cellulose | 21.6 mg |
| Magnesium stearate | 7.2 mg |
| Total | 720 mg |

Powders of each of the components listed above can be mixed well followed by compressing and molding 720 g each into tablets. These tablets may be coated with sugar or a film as necessary.

### [BRIEF DESCRIPTION OF THE DRAWINGS]

[Fig. 1] This is a diagram showing the effects of promoting elimination of blood lactate by 5-{4-[(6-methoxy-1-methyl-1H-benzimidazol-2-yl)methoxy]benzyl}thiazolidine-2,4-dione hydrochloride (Compound B) and the effects of improving increases in blood lactate levels by concomitant administration of metformin (Compound A) and Compound B (Test Example 1).

## Claims

1. A pharmaceutical composition for lowering blood lactate levels, comprising a PPARγ agonist as an active ingredient thereof.

2. A pharmaceutical composition for inhibiting increases in blood lactate levels by promoting elimination of blood lactate, comprising a PPARγ agonist as an active ingredient thereof.

3. A pharmaceutical composition for preventing accumulation of lactate in the blood by promoting elimination of blood lactate, comprising a PPARγ agonist as an active ingredient thereof.

4. A pharmaceutical composition for inhibiting increases in blood lactate levels comprising a PPARγ agonist in combination with a biguanide agent.

5. The pharmaceutical composition according to any one of Claims 1 to 4, wherein the blood lactate is generated from the action of a biguanide agent.

6. The pharmaceutical composition according to any one of Claims 1 to 5, wherein the PPARγ agonist is pioglitazone, rosiglitazone, MCC-555, BMS-298585, AZ-242, LY-519818, R-483, 3-(2,4-dichlorobenzyl)-2-methyl-N-(pentylsulfonyl)-3H-benzimidazole-5-carboxamide, MBX-102, AMG-131, 5-[4-(6-methoxy-1-methyl-1H-benzimidazol-2-ylmethoxy)benzyl]thiazolidine-2,4-dione or a pharmaceutically acceptable salt thereof.

7. The pharmaceutical composition according to any one of Claims 1 to 5, wherein the PPARγ agonist is a thiazolidinedione PPARγ agonist.

8. The pharmaceutical composition according to any one of Claims 1 to 5, wherein the PPARγ agonist is pioglitazone, rosiglitazone or a pharmaceutically acceptable salt thereof.

9. The pharmaceutical composition according to any one of Claims 1 to 5, wherein the PPARγ agonist is 5-[4-(6-methoxy-1-methyl-1H-benzimidazol-2-ylmethoxy)benzyl]thiazolidine-2,4-dione or a pharmaceutically acceptable salt thereof.

10. The pharmaceutical composition according to any one of Claims 1 to 9, wherein the biguanide agent is metformin, fenformin or buformin.

11. The pharmaceutical composition according to any one of Claims 1 to 9, wherein the biguanide agent is metformin.

12. The pharmaceutical composition according to any one of Claims 1 to 11 for preventing or treating diabetes.

13. Use of a PPARγ agonist for manufacturing a blood lactate lowering agent.

14. A kit for preventing adverse side effects occurring due to the action of a biguanide agent in the treatment of diabetes, wherein (a) a first component is a pharmaceutical composition comprising a PPARγ agonist as an active ingredient thereof, and (b) a second component is a pharmaceutical composition comprising a biguanide agent as an active ingredient thereof.

15. The kit according to Claim 14, wherein the adverse side effect occurring due to the action of the biguanide agent is an increase in blood lactate.

16. The kit according to Claim 14 or 15 that is packaged for oral administration.

17. Use of a biguanide agent and a PPARγ agonist for manufacturing a kit for preventing adverse side effects occurring due to the action of a biguanide agent in the treatment of diabetes, wherein (a) a first component is a pharmaceutical composition comprising a PPARγ agonist as an active ingredient thereof, and (b) a second component is a pharmaceutical composition comprising a biguanide agent as an active ingredient thereof.

18. A method for treating diabetes that alleviates adverse side effects caused by a biguanide agent, comprising: concomitant administration to a patient of (a) a pharmaceutical composition comprising a PPARγ agonist as an active ingredient thereof, and (b) a pharmaceutical composition comprising a biguanide agent as an active ingredient thereof.

19. A method for treating diabetes that alleviates adverse side effects caused by a biguanide agent, comprising: concomitant administration to a patient of (a) a pharmaceutical composition comprising a PPARγ agonist as an active ingredient thereof, followed by (b) a pharmaceutical composition comprising a biguanide agent as an active ingredient thereof.

20. The treatment method according to Claim 18 or 19, wherein the adverse side effect caused by the biguanide agent is accumulation of blood lactate.
